# EUROPEAN PATENT SPECIFICATION

(11) **EP 3 216 346 B1**
(45) Date of publication and mention of the grant of the patent: **28.07.2021**
(21) Application number: 15856677.8
(22) Date of filing: 02.11.2015
(51) Int. Cl.: A01N 65/08, A01N 63/14, A23B 7/08, A61K 36/00, A61K 35/644, A01P 1/00

(54) **ANTI-BACTERIAL AGENT**
ANTIBAKTERIELLES MITTEL
AGENT ANTIBACTÉRIEN

(30) Priority: 04.11.2014 JP 2014224252
(43) Date of publication of application: 13.09.2017
(73) Proprietor: Yamada Bee Company, Inc., Tomata-gun, Okayama 708-0393 (JP)
(72) Inventor: WATANABE Tami, Tomata-gun Okayama 708-0393 (JP); SADO Tetuya, Tomata-gun Okayama 708-0393 (JP)
(74) Representative: Grünecker Patent- und Rechtsanwälte PartG mbB
(86) International application number: PCT/JP2015/080901
(87) International publication number: WO 2016/072377

(56) References cited:
- JP-A- H11 199 433
- DINKO HRISTOV DINKOV: "CORRELATION BETWEEN ANTIBACTERIAL AND ANTIOXIDANT ACTIVITY IN OAK HONEYDEW AND ACACIA (ROBINIA PSEUDOACACIA L.), BEE HONEYS", THE V INTERNATIONAL SCIENTIFIC AND PRACTICAL CONFERENCE ON "CURRENT STATE AND PERSPECTIVES OF FOOD INDUSTRY AND CATERING DEVELOPMENT", vol. 1, 1 October 2011 (2011-10-01), pages 265-277, XP055469621, Chelyabinsk, Russia
- JURAJ MAJTAN ET AL: "Honeydew honey as a potent antibacterial agent in eradication of multi-drug resistant Stenotrophomonas maltophilia isolates from cancer patients", PHYTOTHERAPY RESEARCH., vol. 25, no. 4, 29 September 2010 (2010-09-29), pages 584-587, XP055469699, GB ISSN: 0951-418X, DOI: 10.1002/ptr.3304
- DINKO HRISTOV DINKOV: "Quality parameters of Bulgarian kinds of bee honey", MACEDONIAN VETERINARY REVIEW, vol. 37, no. 1, 1 January 2014 (2014-01-01), pages 35-41, XP055469703, DOI: 10.14432/j.macvetrev.2014.01.007
- SHERLOCK ET AL: 'Orla, Comparison of the antimicrobial activity of Ulmo honey from Chile and Manuka honey against methicillin-resistant Staphylococcus aureus, Escherichia coli and Pseudomonas aeruginosa' BMC COMPLEMENTARY AND ALTERNATIVE MEDICINE vol. 10, no. 1, 2010, pages 1 - 5, XP021076661 DOI: 10.1186/1472-6882-10-47
- LUSBY, P.E. ET AL.: 'Bactericidal Activity of Different Honeys against Pathogenic Bacteria' ARCHIVES OF MEDICAL RESEARCH vol. 36, no. 5, 01 September 2005, pages 464 - 467, XP005024627
- GEORGIEV, G. ET AL.: 'HONEYDEW PRODUCERS IN OAK FORESTS OF STRANDZHA MOUNTAIN' SILVA BALCANICA vol. 9, no. 1, 01 January 2008, BULGARIA , SILVA BALCANICA, pages 85 - 90, XP055439683
- Lucía Vela ET AL: "Antioxidant capacity of Spanish honeys and its correlation with polyphenol content and other physicochemical properties", JOURNAL OF THE SCIENCE OF FOOD AND AGRICULTURE, vol. 87, no. 6, 30 April 2007 (2007-04-30) , pages 1069-1075, XP055612536, GB ISSN: 0022-5142, DOI: 10.1002/jsfa.2813
- Werner Von Der Ohe ET AL: "PROGRAM AND ABSTRACTS", 1st World Honeydew Honey Symposium, Tzarevo, Bulgaria, 18 August 2008 (2008-08-18), pages 1-34, XP055706300,

## Description

### Technical Field

The present invention relates to an anti-bacterial agent.

### Background Art

Honey is not only provided for food, but also is utilized as various uses by taking advantages of its anti-bacterial activity. For example, wound healing compositions, cosmetic compositions, and others, which contain honey, have been studied (Patent Literatures 1 and 2)

Manuka (*Leptospermum scoparium*) is a woody plant that mainly grows in New Zealand and that belongs to *Myrtaceae.* Among the types of honeys, honey derived from Manuka is particularly known to have high anti-bacterial activity and is utilized as a medical use, including an agent for treating periodontitis (Patent Literature 3).
D. Dinkov, "CORRELATION BETWEEN ANTIBACTERIAL AND ANTIOXIDANT ACTIVITY IN OAK HONEYDEW AND ACACIA (ROBINIA PSEUDOACACIA L.), BEE HONEYS", The V International Scientific and Practical Conference on "CURRENT STATE AND PERSPECTIVES OF FOOD INDUSTRY AND CATERING DEVELOPMENT", Chelyabinsk, Russia, October 2011, vol. 1, pages 265 - 277 describes the antibacterial and antioxidant activity of oak honeydew and other honeys.
J. Majtan et al., "Honeydew honey as a potent antibacterial agent in eradication of multi-drug resistant Stenotrophomonas maltophilia isolates from cancer patients", Phytother. Res.2011, vol. 25, no. 4, pages 584 - 587 studies the antibacterial activity of honeydew honey compared to manuka honey.
D. Dinkov, "Quality parameters of Bulgarian kinds of bee honey", Macedonian Veterinary Review, January 2014, vol. 37, no. 1, pages 35 - 41 describes quality parameters of Bulgarian bee he honeys.
JP H11 199433 discloses the use of honey as a medicament for reducing the adhesion of microorganisms to the skin in cosmetic compositions.
O. Sherlock et al., "Comparison of the antimicrobial activity of Ulmo honey from Chile and Manuka honey against methicillin-resistant Staphylococcus aureus, Escherichia coli and Pseudomonas aeruginosa", BMC Complementary & Alternative Medicine 2010, 10, pages 1 - 5 study the antimicrobial properties of different types of honey against selected strains of bacteria and a possible alternative therapy for wound healing.
P.E. Lusby et al., "Bactericidal Activity of Different Honeys against Pathogenic Bacteria", Archives of Medical Research, 2005, vol. 36, no. 5, pages 464 - 467 describe the use of honey for therapeutic purposes including treatment of infected wounds and study the antibacterial activity of three locally produced honeys and compared them to commercial therapeutic honeys.
G. Georgiev et al., "HONEYDEW PRODUCERS IN OAK FORESTS OF STRANDZHA MOUNTAIN", Silva Balcanica, 2008, vol. 9, no. 1, pages 85 - 90 describe different types of honeydew honey produced in Strandzha Mountain.
L. Vela et al., "antioxidant capacity of Spanish honey and its correlation with polyphenol content and other physicochemical properties",J. Sci. Food Agric. 2007, 87, pages 1069 - 1075 study the radical-scavenging capacity of different honeys.
Program and Abstracts, 1st World Honeydew Honey Symposium, Tzarevo, Bulgaris, 18. August 2008, pages 23 - 24 describes the antibacterial activity of Bulgarian honeydew and blossom honeys and the use of honeydew honeys in therapy. B. Mamdouh in his PhD thesis for Sheffield University in 2014, titled "IN VITRO-STUDIES RELATING TO HONEY AS AN ALTERNATIVE APPROACH TO WOUND THERAPY" carried out studies into the antibacterial activity of a range of commerically available honeys, and found that Manuka honey had higher activity against *Pseudomonas aeruginosa* than an oak honey (Table 7).

### Citation List

### Patent Literature

Patent Literature 1: JP 2003-516357 T
Patent Literature 2: JP 2003-63984 A
Patent Literature 3: JP 2007-277210 A

### Summary of Invention

### Technical Problem

Since honey has been provided for food for a long time, it can be ingested without problems and can be utilized as anti-bacterial agents in a variety of uses. The honey derived from Manuka, which is known to have high anti-bacterial activity, chiefly displays high anti-bacterial activity against *Staphylococcus aureus;* however, its anti-bacterial activities against other bacteria cannot be said to be sufficient.

An object of the present invention is to provide an anti-bacterial agent with excellent anti-bacterial activity comprising honey as an effective ingredient.

### Solution to Problem

The present inventors have found that honey derived from *Quercus hartwissiana* has very excellent anti-bacterial activity. The present invention provides an anti-bacterial agent comprising a honey for use in medicine, wherein the nectar source of the honey is a nectar or sap of *Quercus hartwissiana,* a secretion of the insect which is parasitic on *Quercus hartwissiana,* or a combination thereof, wherein the anti-bacterial agent has anti-bacterial activity against *Pseudomonas aeruginosa,* and wherein the content of the honey of which *Quercus hartwissiana* is the nectar source is 100% by mass, based on the solid content of the anti-bacterial agent.

The present invention further provides the non-therapeutic use of an anti-bacterial agent comprising a honey, wherein the nectar source of the honey is a nectar or sap of *Quercus hartwissiana,* a secretion of the insect which is a parasitic on *Quercus hartwissiana,* or a combination thereof, in a food, wherein the anti-bacterial agent has anti-bacterial activity against *Pseudomonas aeruginosa,* and wherein the content of the honey of which *Quercus hartwissiana* is the nectar source is 100% by mass or more, based on the solid content of the anti-bacterial agent

The anti-bacterial agent further has anti-bacterial activity against at least one selected from the group consisting of *Staphylococcus aureus,* drug-resistant *Staphylococcus aureus,* and *Escherichia coli.*

### Advantageous Effects of Invention

The anti-bacterial agent according to the present invention has excellent anti-bacterial activity.

### Description of Embodiments

The anti-bacterial agent of the present invention comprises honey, wherein the nectar source of the honey is *Quercus hartwissiana.*

Specifically, the honey of which *Quercus hartwissiana* is the nectar source refers to honey of which a nectar or sap of *Quercus hartwissiana*, a secretion of the insect which is parasitic on *Quercus hartwissiana*, or a combination thereof is the nectar source. *Quercus hartwissiana* is a woody plant that mainly grows in from Europe to the periphery of the Black Sea and a part of Asia naturally and that belongs to *Fagaceae* in *Quercus.* It is possible to assume the nectar source of honey, for example, by analyzing the morphological characteristics of pollen contained in the honey through microscopic observation or by analyzing the sequence information of DNAs contained in pollen.

The honey of which *Quercus hartwissiana* is the nectar source can be obtained by carrying out apiculture using *Quercus hartwissiana* as a nectar source and collecting the honey according to a method known in the art. The honey is such that only *Quercus hartwissiana* is the nectar source. For example, if beehives are provided in the growing area of *Quercus hartwissiana,* the principle nectar source of the honey can be made *Quercus hartwissiana.* Also, if beehives and the vegetation of *Quercus hartwissiana* are, for example, arranged to coexist within an enclosed space such as a house, the method allows *Quercus hartwissiana* to occupy the entire region where the bees collect honey.

The honey may be that which is collected intact; or it may be that which has undergone the removal of proteins, waxes, dusts, or remains of the nest, purification treatments such as filtration, or treatments including sterilization, drying or concentration. The types of bees that are utilized to collect the honey are not particularly limited.

In the anti-bacterial agent, the content of the honey of which *Quercus hartwissiana* is the nectar source is 100% by mass based on the solid content of the anti-bacterial agent.

The anti-bacterial agent may further comprise other ingredients having anti-bacterial activity that are known in the art, in addition to the honey. The other ingredients having anti-bacterial activity are preferably natural anti-bacterial ingredients, such as chitin, chitosan, and catechin, which can be ingested. When the anti-bacterial gent is used in the utility that is not for oral intake, it may comprise the other ingredients having anti-bacterial activity, including anti-bacterial ingredients of the organic type and anti-bacterial ingredients of the inorganic type.

The anti-bacterial agent according to the present embodiment has excellent anti-bacterial activity. The anti-bacterial activity refers to the activity which inhibits the proliferation of bacteria. The bacteria against which the anti-bacterial activities are displayed are not particularly limited. However, it is preferable that the anti-bacterial agent according to the present embodiment further has anti-bacterial activity against at least one species selected from the group consisting of *Staphylococcus aureus,* drug-resistant *Staphylococcus aureus,* and *Escherichia coli*; and it is more preferable that the anti-bacterial agent has anti-bacterial activities against all species of *Staphylococcus aureus,* drug-resistant *Staphylococcus aureus, Pseudomonas aeruginosa,* and *Escherichia coli.* The anti-bacterial agent according to the present embodiment particularly has high anti-bacterial activity against *Escherichia coli* and *Pseudomonas aeruginosa.* Manuka honey that is known as honey with high anti-bacterial activity is known to display anti-bacterial activity against *Staphylococcus aureus;* however, the anti-bacterial agent according to the present embodiment is advantageous from the standpoint that it displays high anti-bacterial activities against all of the four species of the bacteria mentioned above.

The anti-bacterial agent according to the present embodiment shows very high anti-bacterial activity and it has anti-bacterial activity against species of the bacteria, wherein the activity is equivalent to or greater than the Manuka honey that is known as honey having high anti-bacterial activity. The extent of the anti-bacterial activity of the Manuka honey is customarily expressed as various values such as the mg quantity of methylglyoxal (MGO) in one kg honey or the concentration of an aqueous phenol solution that displays the same anti-bacterial activity against *Staphylococcus aureus* as does the honey. For example, the anti-bacterial agent according to the present embodiment has higher anti-bacterial activity than the commercial Manuka honey having at least 300 mg/kg, 450 mg/kg, or 790 mg/kg of methylglyoxal concentration. The methylglyoxal concentration in honey can, for example, be measured with HPLC using as solvent, a 0.05% (w/v) o-phenylenediamine aqueous solution. The anti-bacterial agent according to the present embodiment can be such that its anti-bacterial activity against one species selected from the group consisting of *Staphylococcus aureus,* drug-resistant *Staphylococcus aureus, Pseudomonas aeruginosa,* and *Escherichia coli* is higher than that of the Manuka honey having at least 450 mg/kg of methylglyoxal concentration; and it can be such that its anti-bacterial activity against *Escherichia coli* and/or *Pseudomonas aeruginosa* is higher than that of the Manuka honey having at least 790 mg/kg of methylglyoxal concentration.

The anti-bacterial activity of the anti-bacterial agent according to the present embodiment against *Staphylococcus aureus* is preferably 4.0 % by mass or more and is more preferably 5.0 % by mass or more, in terms of the phenol concentration equivalent. The anti-bacterial activity of the anti-bacterial agent against *Escherichia coli* is preferably 180 mg/ml or less, more preferably 120 mg/ml or less, further more preferably 80 mg/ml or less, and particularly preferably 60 mg/ml or less, in terms of the minimum inhibitory concentration. The anti-bacterial activity of the anti-bacterial agent against drug-resistant *Staphylococcus aureus* is preferably 120 mg/ml or less, more preferably 80 mg/ml or less, and further more preferably 60 mg/ml or less, in terms of the minimum inhibitory concentration. The anti-bacterial activity of the anti-bacterial agent against *Pseudomonas aeruginosa* is preferably 180 mg/ml or less, more preferably 120 mg/ml or less, and further more preferably 90 mg/ml or less.

The anti-bacterial agent according to the present embodiment may be that which is only comprised of the effective ingredient of honey ,wherein the nectar source of the honey is *Quercus hartwissiana*; it may comprise other ingredients insofar as its anti-bacterial effects are not hindered. Examples of the other ingredients include pharmaceutically acceptable additives (such as an excipient, a binder, a lubricant, a disintegrator, an emulsifier, a surfactant, a base, a solubilizer, and a suspending agent).

The anti-bacterial agent according to the present embodiment may be in any shape of solid, liquid, paste, , and it may be in the form of tablets (which include an uncoated tablet, a sugar-coated tablet, a foam tablet, a film-coated tablet, a chewable tablet, a troche, and others), a capsule, a pill, a powder (powdered drug), a fine granule, a granule, a liquid drug, a suspension, an emulsion, a syrup, a paste, an injection (including a case where it is combined with distilled water or an infusion, such as an amino acid infusion or an electrolyte infusion, to prepare a liquid drug when using), or a spray. These various formulations can be prepared, for example, by admixing the honey and the other ingredients to form the aforementioned dosage forms.

The anti-bacterial agent according to the present embodiment can be used in the utility of foods, medicines, and others.

The anti-bacterial agent according to the present embodiment may be combined with other food materials to be utilized as food compositions comprising said anti-bacterial agent. The food compositions include any food composition that an animal, including a human, can ingest. Since the anti-bacterial agent according to the present embodiment has excellent anti-bacterial activity, food compositions with superior preservability can be made by allowing the food compositions to comprise the anti-bacterial agent.

Minerals, vitamins, flavonoids, quinones, polyphenols, amino acids, nucleic acids, essential fatty acids, an algefacient, a binder, a sweetener, a disintegrating agent, a lubricant, a coloring agent, a flavoring agent, a stabilizer, an antiseptic, a release adjusting agent, a surfactant, a dissolution agent, a moistening agent, and others can be incorporated to the food composition if necessary.

Examples of food compositions include beverages (refreshing drinks such as coffee, juice, and a tea drink, a milk drink, a lactic fermenting drink, a yogurt drink, a carbonated drink, liquors such Japanese sake, a Western liquor, a fruit liquor, and a honey liquor); spreads such as custard cream; pastes such as a fruit paste; Western confectionaries (chocolate, a doughnut, a pie, a cream puff, gum, jelly, a candy, a cookie, a cake, a pudding, and the like); Japanese confectionaries (a Daifuku (a rice cake stuffed with red bean paste), a rice cake, a steamed bun, a Castella sponge cake, an Anmitsu (an agar jelly served with red bean paste and brown sugar syrup), a sweet bean jelly, and the like); edible ices (ice cream, ice candy, and sherbet); foods (curry, beef bowel, porridge of rice, miso soup, soup, meat sauce, pasta, a pickle, jam, honey, royal jelly, and propolis); and seasonings (a dressing, a condiment to be sprinkled over rice, a flavor enhancer, and a soup mix). The anti-bacterial agent may be made into a food composition consisting only of said anti-bacterial agent.

The method for producing the food composition can appropriately follow those which are known in the art. For example, the honey can be mixed into an intermediate or final product during the production process for a food composition, whereby there can be obtained the food composition to be used for the purposes mentioned above.

The food composition can also be used as a health food, a functional food, a dietary supplement, a supplement, or a food for specified health use, each of which has anti-bacterial activity. When used as a supplement, its unit dosage form is not particularly limited and can be appropriately selected. The examples include a tablet, a capsule, a granule, a liquid, and a powder.

The anti-bacterial agent according to the present example can be utilized in the form of pharmaceuticals (particularly, an oral pharmaceutical). When it is prepared into a pharmaceutical composition, the anti-bacterial agent can be prepared, together with a pharmaceutically acceptable non-toxic carrier, a diluent or an excipient, into forms of tablets (which include an uncoated tablet, a sugar-coated tablet, a foam tablet, a film-coated tablet, a chewable tablet, a troche, and others), a capsule, a pill, a powder (powdered drug), a fine granule, a granule, a liquid drug, a suspension, an emulsion, a syrup, a paste, an injection (including a case where it is combined with distilled water or an infusion, such as an amino acid infusion or an electrolyte infusion, to prepare a liquid drug when using), each of which can be a pharmaceutical formulation.

### EXAMPLES

The present invention will be described in more detail by way of the examples below.

### (Test Example 1)

Honey diluted to 50 w/v % was added to a bacterium-culturing medium and the anti-bacterial activity of each species of honeys against *Staphylococcus aureus subsp. aureus* (NBRC 13276) was confirmed by the method in which said activity is calculated as being converted to an aqueous phenol concentration, using a disk diffusion method where anti-bacterial activities were compared in terms of the sizes of the zone of inhibition to be formed. Specifically, an aperture having a diameter of 8 mm was opened in the center of a solidified Muller Hinton II agar medium that contained the *Staphylococcus aureus.* The sample (honey) with a concentration of 50% was inoculated in the aperture and was cultured at 37 °C for 18 hours.

After culturing, the diameter of the zone of inhibition was measured. The phenol concentration equivalent (% by mass) of the sample (honey) was calculated based on the correlation between the phenol concentration and the diameter of the zone of inhibition when phenol was used as a sample. The honeys employed honey of which *Quercus hartwissiana* is the nectar source that had been obtained from Alexander Co. Ltd. (Example 1) and Manuka honeys having 302.3 mg/kg (trade name: Manuka honey UMF10+ from Comvita Japan Co. Ltd.), 458.5 mg/kg (trade name: Manuka honey from Sojitz Food Corporation), and 790.9 mg/kg (trade name: strong Manuka honey from TCN Co., Ltd.) of methylglyoxal concentrations in one kg honey, respectively (Comparative Examples 1-3). The methylglyoxal concentration in each honey was measured using HPLC with a 0.05% (w/v) o-phenylenediamine aqueous solution as solvent. Results are shown in Table 1. The phenol concentration equivalent represents that as its value grows larger, the anti-bacterial activity becomes higher.

### (Test Example 2)

The minimum inhibitory concentration (MIC) method was used to confirm the anti-bacterial activity of each types of the honeys against *Escherichia coli* (ATCC 10536), drug-resistant (penicillin-resistant) *Staphylococcus aureus* (ATCC 6538P), and *Pseudomonas aeruginosa* (ATCC 43504). Specifically, the sample (honey) was diluted with a CAMHB culture medium so that its concentration was from 53 to 900 mg/mL, and each bacterial solution was inoculated in the medium. After culturing at 36 °C for 20 hours, the growth state of each species of the bacteria was confirmed and the minimum inhibitory concentration was evaluated. Results are shown in Table 1. The minimum inhibitory concentration represents that as its value grows lower, the anti-bacterial activity becomes higher.

**[Table 1]**

| | | Methylglyoxal concertation (mg/kg) | Test Example 1 Phenol concentration Equivalent (% by mass) | Test Example 2 Minimum inhibitory concentration (mg/ml) | | |
|---|---|---|---|---|---|---|
| | | | Anti-*Staphylococcus aureus* | Anti-*Escherichia coli* | Anti-drug-resistant *Staphylococcus aureus* | Anti-*Pseudomonas aeruginosa* |
| Comparative Example 1 | Manuka honey | 790.9 | 5.7 | 79 | 53 | 119 |
| Comparative Example 2 | Manuka honey | 458.5 | 4.3 | 119 | 79 | 119 |
| Comparative Example 3 | Manuka honey | 302.3 | - | 178 | 119 | 178 |
| Example 1 | Honey of which *Quercus hartwissiana* is the nectar source | | 5.3 | 53 | 53 | 79 |

As the results of the anti-bacterial activity tests, the honey of which *Quercus hartwissiana* is the nectar source displayed higher anti-bacterial activity against *Escherichia coli* and *Pseudomonas aeruginosa* as well as displays the equivalent anti-bacterial activity against drug-resistant *Staphylococcus aureus* as compared with the Manuka honey having methylglyoxal concentration of 790.9 mg/kg; it displayed higher anti-bacterial activity against *Staphylococcus aureus* than the Manuka honey having methylglyoxal concentration of 458.5 mg/kg.

## Claims

1. An anti-bacterial agent comprising a honey for use in medicine, wherein the nectar source of the honey is a nectar or sap of *Quercus hartwissiana,* a secretion of the insect which is parasitic on *Quercus hartwissiana,* or a combination thereof, wherein the anti-bacterial agent has anti-bacterial activity against *Pseudomonas aeruginosa,* and wherein the content of the honey of which *Quercus hartwissiana* is the nectar source is 100% by mass, based on the solid content of the anti-bacterial agent.

2. The anti-bacterial agent according to claim 1, further having anti-bacterial activity against at least one selected from the group consisting of *Staphylococcus aureus* and drug-resistant *Staphylococcus aureus.*

3. Non-therapeutic use of an anti-bacterial agent comprising a honey, wherein the nectar source of the honey is a nectar or sap of *Quercus hartwissiana,* a secretion of the insect which is a parasitic on *Quercus hartwissiana,* or a combination thereof, in a food, wherein the anti-bacterial agent has anti-bacterial activity against *Pseudomonas aeruginosa,* and wherein the content of the honey of which *Quercus hartwissiana* is the nectar source is 100% by mass or more, based on the solid content of the anti-bacterial agent.

4. The use according to claim 3, wherein the anti-bacterial agent further has anti-bacterial activity against at least one selected from the group consisting of *Staphylococcus aureus,* drug-resistant *Staphylococcus aureus,* and *Escherichia coli.*

5. The use according to claim 3 or 4 for improving preservability of the food composition.

6. The use according to any one of claims 3 to 5 as a health food, a functional food, a dietary supplement, a supplement or a food for specified health use, each of which has anti-bacterial activity.

## Patentansprüche

1. Antibakterielles Mittel, umfassend einen Honig zur Verwendung in der Medizin, wobei die Nektarquelle des Honigs ein Nektar oder Saft von *Quercus hartwissiana,* ein Sekret des Insekts, das ein Parasit auf *Quercus hartwissiana* ist, oder eine Kombination davon ist, wobei das antibakterielle Mittel eine antibakterielle Aktivität gegen *Pseudomonas aeruginosa* aufweist, und wobei der Gehalt des Honigs, dessen Nektarquelle *Quercus hartwissiana* ist, 100 Massenprozent beträgt, bezogen auf den Feststoffgehalt des antibakteriellen Mittels.

2. Antibakterielles Mittel nach Anspruch 1, das ferner eine antibakterielle Aktivität gegen mindestens eines, ausgewählt aus der Gruppe bestehend aus *Staphylococcus aureus* und arzneimittelresistentem *Staphylococcus aureus,* aufweist.

3. Nicht-therapeutische Verwendung eines antibakteriellen Mittels, umfassend einen Honig, wobei die Nektarquelle des Honigs ein Nektar oder Saft von *Quercus hartwissiana,* ein Sekret des Insekts, das ein Parasit auf *Quercus hartwissiana* ist, oder eine Kombination davon ist, in einem Lebensmittel, wobei das antibakterielle Mittel eine antibakterielle Aktivität gegen *Pseudomonas aeruginosa* aufweist, und wobei der Gehalt des Honigs, dessen Nektarquelle *Quercus hartwissiana* ist, 100 Massenprozent oder mehr beträgt, bezogen auf den Feststoffgehalt des antibakteriellen Mittels.

4. Verwendung nach Anspruch 3, wobei das antibakterielle Mittel ferner antibakterielle Aktivität gegen mindestens eines, ausgewählt aus der Gruppe bestehend aus *Staphylococcus aureus,* arzneimittelresistentem *Staphylococcus aureus* und *Escherichia coli,* aufweist.

5. Verwendung nach Anspruch 3 oder 4 zur Verbesserung der Konservierbarkeit der Lebensmittelzusammensetzung.

6. Verwendung nach einem der Ansprüche 3 bis 5 als gesundes Lebensmittel, funktionelles Lebensmittel, diätetisches Ergänzungsmittel, Nahrungsergänzungsmittel oder Lebensmittel mit spezifizierter gesundheitlicher Verwendung, wobei jedes davon antibakterielle Aktivität aufweist.

## Revendications

1. Agent antibactérien comprenant du miel pour l'utilisation en médecine, la source de nectar du miel étant un nectar ou de la sève de *Quercus hartwissiana,* une sécrétion de l'insecte qui est un parasite de *Quercus hartwissiana,* ou une combinaison de ceux-ci, l'agent antibactérien ayant une activité antibactérienne contre *Pseudomonas aeruginosa,* et la teneur du miel dont *Quercus hartwissiana* est la source de nectar étant de 100 % en masse, sur la base de la teneur en matières solides de l'agent antibactérien.

2. Agent antibactérien selon la revendication 1, ayant en outre une activité antibactérienne contre au moins l'un sélectionné dans le groupe constitué de *Staphylococcus aureus* et *Staphylococcus aureus* résistant au médicament.

3. Utilisation non thérapeutique d'un agent antibactérien comprenant un miel, la source de nectar du miel étant un nectar ou de la sève de *Quercus hartwissiana,* une sécrétion de l'insecte qui est un parasite de *Quercus hartwissiana,* ou une combinaison de ceux-ci, dans un aliment, l'agent antibactérien ayant une activité antibactérienne contre *Pseudomonas aeruginosa,* et la teneur du miel dont *Quercus hartwissiana* est la source de nectar étant de 100 % en masse ou plus, sur la base de la teneur en matières solides de l'agent antibactérien.

4. Utilisation selon la revendication 3, l'agent antibactérien ayant en outre une activité antibactérienne contre au moins l'un sélectionné dans le groupe constitué de *Staphylococcus aureus, Staphylococcus aureus* résistant au médicament, et *Escherichia coli.*

5. Utilisation selon la revendication 3 ou 4 pour améliorer l'aptitude à la conservation de la composition alimentaire.

6. Utilisation selon l'une quelconque des revendications 3 à 5 comme aliment de santé, aliment fonctionnel, complément alimentaire, complément ou aliment pour une utilisation spécifiée dans la santé, chacun ayant une activité antibactérienne.
